(19) Europäisches Patentamt
European Patent Office
Office européen des brevets

(11) **EP 4 185 446 B1**

(12) **EUROPEAN PATENT SPECIFICATION**

(45) Date of publication and mention of the grant of the patent:
**14.02.2024 Bulletin 2024/07**

(21) Application number: **22744774.5**

(22) Date of filing: **25.07.2022**

(51) International Patent Classification (IPC):
***B26B 19/38*** (2006.01)

(52) Cooperative Patent Classification (CPC):
**B26B 19/388**

(86) International application number:
**PCT/EP2022/070731**

(87) International publication number:
**WO 2023/016783 (16.02.2023 Gazette 2023/07)**

(54) **POWERING A FUNCTIONAL UNIT AND OBTAINING A MEASUREMENT SIGNAL VIA A SINGLE PAIR OF ELECTRICAL CONNECTION PATHS**

STROMVERSORGUNG EINER FUNKTIONSEINHEIT UND ERHALTUNG EINES MESSSIGNALS ÜBER EIN EINZIGES PAAR VON ELEKTRISCHEN VERBINDUNGSWEGEN

ALIMENTATION D'UNITÉ FONCTIONNELLE ET OBTENTION D'UN SIGNAL DE MESURE PAR L'INTERMÉDIAIRE D'UNE SEULE PAIRE DE CHEMINS DE CONNEXION ÉLECTRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.08.2021 EP 21191197**

(43) Date of publication of application:
**31.05.2023 Bulletin 2023/22**

(73) Proprietor: **Koninklijke Philips N.V.**
**5656 AG Eindhoven (NL)**

(72) Inventors:
 • **BISSCHOP, Oedilius, Johannes**
 **5656 AG Eindhoven (NL)**
 • **ALGRA, Sietze**
 **5656 AG Eindhoven (NL)**
 • **MOYO, Fortune**
 **5656 AG Eindhoven (NL)**
 • **SEMENOV, Andrii**
 **5656AG Eindhoven (NL)**

(74) Representative: **Philips Intellectual Property & Standards**
**High Tech Campus 52**
**5656 AG Eindhoven (NL)**

(56) References cited:
**WO-A1-2015/135682     US-A- 4 594 777**
**US-A1- 2012 024 552**

**Description**

FIELD OF THE INVENTION

This disclosure relates to a device, such as a shaving device, comprising a main body and a functional unit comprising a functional member and a sensing component, and in particular to an arrangement whereby the functional member can be powered and a measurement signal can be obtained from the sensing component using a single pair of electric connection paths between the main body and the functional unit.

BACKGROUND OF THE INVENTION

A recent focus in the field of personal care devices, and in particular facial hair shaving devices, is the provision of an enhanced sensorial experience to the user of such a device alongside the performance of one or more personal care functions, such as shaving, hair removal, skin massage, etc.

For example, an enhanced sensorial experience may be provided to the user of a personal care device via the use of an infra-red (IR) or Near IR (NIR) light module within the personal care device. The IR or Near IR light module may be powered to provide gentle warmth to the user and/or treat the skin of the user while the personal care device is in use performing the personal care function(s).

It will be appreciated that in this example, the sensorial experience provided by the IR or NIR light module will improve with the proximity of the IR or NIR light module to the skin of the user (when the personal care device is in use). In view of this, the IR or NIR light module may be provided within a functional unit of the personal care device, with the functional unit being the part of the personal care device that provides one or more personal care functions such as shaving or hair cutting.

Often, the functional unit may be removable from a main body of the personal care device to facilitate cleaning or replacement of the functional unit, for example to clean debris from the hair cutting elements or to replace the hair cutting elements altogether. Therefore the functional unit requires electrical connections to the main body to receive power.

Given the environment in which personal care devices are used, the coupling between the main body and the functional unit needs to be waterproof, which makes the construction of the main body and functional unit complex.

Furthermore, it may also be desirable to measure certain parameters relating to the operation of the functional unit, for example, a temperature of the functional unit or light module, to enhance user experience and/or ensuring user safety. The measurements of this/these parameters need to be provided from the functional unit to the main body for evaluation, but in view of the complexity required to ensure that the personal care device is waterproof it is undesirable to provide additional electrical connections between the main body and functional unit.

An example of prior art is given by the patent documentation WO 2015/135682 A1. Said document discloses an electric motor for a shaving device comprising a load detector and a controller wherein the controller sets the average voltage based on the detected load.

SUMMARY OF THE INVENTION

It is an object of the invention to provide a device comprising a functional unit and a main body, that enables the functional unit to be powered and a measurement signal to be obtained from the functional unit via a single pair of electrical connection paths.

According to a first specific aspect, there is provided a device comprising a main body comprising a power source and control circuitry, and a functional unit comprising a functional member and a sensing component, wherein the functional member and the sensing component are arranged electrically in parallel with one another, and wherein the functional member has a threshold voltage below which the functional member is electrically non-conductive or substantially electrically non-conductive, and above which the functional member is electrically conductive, wherein the control circuitry and the power source are electrically coupled to the functional unit by a single pair of electric connection paths, and wherein the control circuitry and the power source are configured to switch between providing first and second voltages to the functional unit via the single pair of electric connection paths, wherein the first voltage exceeds the threshold voltage and the second voltage is below the threshold voltage to enable a measurement signal to be obtained from the sensing component via the single pair of electric connection paths. This aspect has the advantage that the single pair of electric connection paths may be utilized to both power a functional member, and obtain a measurement signal from a sensing component, thereby reducing the number of electrical connections required between the main body and the functional unit.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 diagrammatically shows an electric shaver according to the invention,
Fig. 2 is a simplified circuit diagram of an exemplary device according to the invention,
Fig. 3 is a circuit diagram of an exemplary device according to the invention,
Fig. 4 is a circuit diagram of an exemplary device according to the invention,
Fig. 5 is a circuit diagram of an exemplary device according to the invention.

DETAILED DESCRIPTION OF EMBODIMENTS

As noted above, the embodiments described herein provide a device comprising a functional unit and a main body, that enables the functional unit to be powered and a measurement signal to be obtained from the functional unit via a single pair of electrical connection paths. The device can be a hand-held device, i.e. a device that is to be held in a hand of a user. The user of the device may be the person that the device is used on (for example, the user is using the device on themselves).

The functional unit comprises a functional member for performing one or more functions with respect to the user. The device can be a personal care device, in which case the functional unit and thus the functional member can perform a function to supplement or enhance a personal care function. For example, the personal care function can be cutting hair, shaving, removing hair, brushing teeth, massaging skin, etc. In these examples, the personal care device can be an electric shaver, an epilator, an electric toothbrush, a skin massager, etc. In some embodiments, the functional member comprises one or more light sources for generating IR or NIR light to warm the skin, increase blood perfusion and/or stimulate blood circulation when the personal care device is being used to perform the personal care function. This may result in a healthier appearance of the skin and/or a re-energized feeling of the skin. It will be appreciated that NIR light may more deeply penetrate the skin than IR light, enabling improved provision of the aforementioned benefits.

In some embodiments, the functional member may comprise one or more light sources for generating visible light to provide skin benefits when the personal care device is being used to perform the personal care function. For example, red wavelengths of visible light may be used to treat skin wounds and/or irritation and/or maintain skin health, blue wavelengths may be used to treat acne, green wavelengths may be used to treat dry skin, and yellow wavelengths may be used to treat age spots.

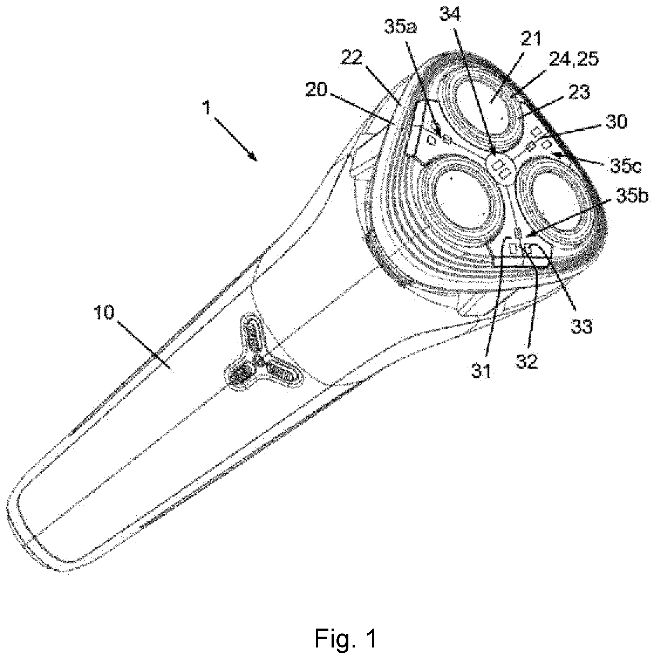

Fig. 1 diagrammatically shows a device in the form of an electric shaver 1 according to a first embodiment of the invention. In the shown example, the electric shaver 1 is of the rotary type and comprises a main body 10 that is intended to be taken hold of by a user of the shaver 1, and a functional unit (specifically, in this illustrated embodiment, a shaving unit) 20 that is intended to contact a portion of skin to be subjected to a shaving action (that is, a personal care function). The main body 10 of the shaver 1 is also commonly referred to as handle, and the shaving unit 20 of the shaver 1 is also commonly referred to as a shaving head. For various reasons such as a need to service and/or clean the shaving unit 20, a need to replace the shaving unit 20 by a unit of another type, etc., it is practical if the shaving unit 20 is removably or hingably mounted to the main body 10. The shaving unit 20 includes a number of hair-cutting units 21, the number being three in the shown example. When the electric shaver 1 is applied for the purpose of subjecting a portion of skin to a shaving action, the actual process of cutting off hairs protruding from the portion of skin takes place at the position of the hair-cutting units 21. For the purpose of supporting the hair-cutting units 21, the shaving unit 20 comprises a base member 22.

Each of the hair-cutting units 21 comprises a combination of an external cutting member 23 that is of a generally cup-shaped design and an internal cutting member (not shown) that is equipped with at least one hair-cutting element and that is at least partially accommodated in the interior of the external cutting member 23. The external cutting member 23 has hair-entry openings 24 in an annular cutting track surface 25. During a shaving action, hairs extending through the hair-entry openings 24 and protruding to the interior of the external cutting member 23 are cut off as soon as they are encountered by a hair-cutting element of the internal cutting member. A shaving action as mentioned can be performed when the internal cutting member is activated to rotate and a portion of skin is actually contacted by the external cutting member 23 at the position of the cutting track surface 25. Activation of the internal cutting member may take place in a known manner by means of a drive mechanism of the shaver 1. This drive mechanism (e.g. a motor), is typically located in the main body 10, and the rotational movement of the motor is transferred to the internal cutting members of the hair-cutting units 21 in the functional unit 20. When the combination of the external cutting member 23 and the internal cutting member is moved over the portion of skin while the internal cutting member is driven to rotate, it is achieved that hairs protruding from the portion of skin are caught in the hair-entry openings 24 of the external cutting member 23 and are cut off in that position.

It is to be noted that the foregoing general information about the electric shaver 1 according to the first embodiment of the invention is applicable to the devices 200, 300, 400 and 500 described below when those devices are implemented as an electric shaver.

In the electric shaver 1 according to the first embodiment of the invention, the shaving unit 20 comprises a skin-heating unit 30 that serves for emitting infrared or near-infrared light to the skin and to heat the skin. The skin-heating unit 30 comprises a skin-contacting member 31 having a skin-contacting surface 32 that is arranged adjacent to the hair-cutting units 21 to contact the skin during a shaving action. Further, the skin-heating unit 30 comprises a functional member in the form of one or more infrared or near-infrared light sources 33, which will hereinafter be simply referred to as light sources 33 for the sake of clarity, and which may comprise LEDs, for example. The light sources 33 are integrated in the skin-contacting member 31. The light sources 33 can be both thermally coupled to the skin-contacting surface 32 to heat the skin-contacting surface 32 and the skin in contact therewith by the thermal energy dissipated by the light sources 33 as a result of their limited electric-to-optical conversion efficiency, and arranged to emit infrared or near-infrared light via the skin-contacting surface 32 to heat the skin by optical absorption of the light by the skin tissue. For example, the skin-contacting member 31 may comprise material that is transparent to the infrared or near-infrared light and the light sources 33 may be embedded in the material. When the functional member of the skin-heating unit 30 is activated during a shaving action, it is achieved that the skin is subjected to more actions besides the shaving action, particular actions in which the skin is stimulated and heated, which may lead to improvement of the condition/appearance of the skin and also to improvement of shaving comfort.

It is noted that the skin-heating unit 30 is configured to actually realize transfer of heat to the skin on the basis of both thermal conduction and optical radiation. In view of the thermal conduction aspect, it is advantageous if the design of the skin-heating unit 30 (or a collection of skin-heating units 30) is chosen so as to enable contact to the skin over an area of considerable size so that heat can be spread and hot spots can be avoided. This implies that it is generally advantageous to have a plurality of light sources 33 in the shaving unit 20. In view of the optical radiation aspect, it is advantageous if the wavelength of the light emitted by the light sources 33 is chosen so as to enable deep skin penetration of the light, so that the skin can really be heated from the inside.

In the shown example, the skin-contacting member 31 is provided in the form of a bracket that is arranged to extend between and partially support the three hair-cutting units 21. According to an advantageous option, the skin-contacting member 31 including the light sources 33 is removably or hingably arranged in the shaving unit 20. In order to have a practical distribution of the infrared or near-infrared light, the light sources 33 are arranged to emit the infrared or near-infrared light via a central section 34 of the skin-contacting surface 32 arranged between the hair-cutting units 21 and via peripheral sections 35a, 35b, 35c of the skin-contacting surface 32 arranged between each of the three different pairs of adjacent hair-cutting units 21 in the equilateral triangular configuration.

Although not shown in Fig. 1, the shaving unit 20 also comprises a sensing component for measuring a parameter and for providing a measurement signal representing the measurement. In some embodiments, the sensing component is provided to measure a parameter relating to the functional member(s) or their operation. For example, in embodiments where the functional member(s) are IR or NIR light sources, the sensing component can measure a parameter relating to the temperature or change in temperature of the light sources, or the temperature or change in temperature of the skin-contacting surface 32 of the skin-heating unit 30, or the temperature or change in temperature of the skin on which the light from the light sources 33 is incident.

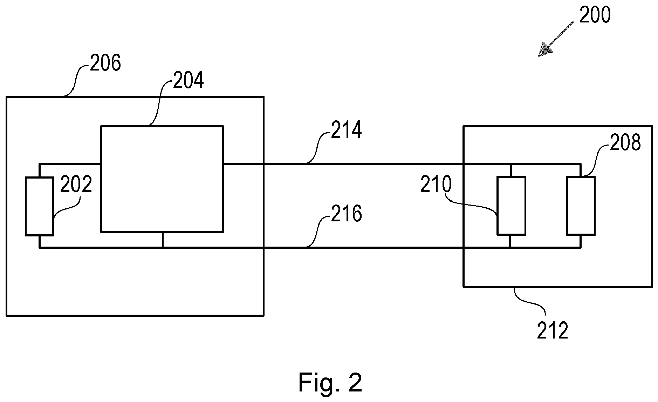

Fig. 2 is a simplified circuit diagram of an exemplary device 200 according to the invention. It will be appreciated that the device 200 may be implemented in the form of the electric shaver 1 described above. Although the device 200 may be implemented as an electric shaver, it will also be appreciated that the device 200 may alternatively be implemented as any of the following: a grooming device, a toothbrush, a depilation device, a photoepilator, etc. Additionally, the device 200 may be implemented as any suitable device comprising a functional unit and a main body, where the functional unit requires power to execute one or more functions, and a measurement signal relating to one or more parameters is to be obtained.

For example, the device 200 may be a personal care device, and the functional unit may be a personal care unit. The personal care device may be an electric shaver (for example, electric shaver 1). Additionally, in some examples, the functional unit may be a shaving unit (for example, shaving unit 20). In some embodiments, the functional unit is releasably coupled to the main body. Additionally, in some examples, the functional member may comprise one or more light emitting diodes (LEDs) arranged in the functional unit to emit light during the personal care operation.

The device 200 comprises a power source 202 (for example, a battery) and control circuitry 204, that are comprised within main body 206. The main body 206 may correspond to the main body 10 of the electric shaver 1 described above.

The device 200 further comprises a functional member 208 and a sensing component 210 comprised within a functional unit 212. The functional member 208 and the sensing component 210 are arranged electrically in parallel with one another between a single pair of electrical connection paths 214, 216. The functional unit 212 may correspond to the shaving unit 20 of the electric shaver 1 described above.

The functional member 208 has a threshold voltage below which the functional member 208 is electrically non-conductive or substantially electrically non-conductive, and above which the functional member 208 is electrically conductive. It will be appreciated that, on application of a voltage that exceeds the threshold voltage of the functional member 208, the functional member 208 will activate and perform the specified function (e.g. emitting light). It will be appreciated that the functional member 208 may comprise one or more elements that, when activated, will provide one or more personal care functions and/or one or more sensorial experiences. For example, the functional member 208 may comprise one or more optical elements (for example, a light-emitting diode (LED)) and/or heating elements (for example, a NIR LED or a resistive heating element).

The control circuitry 204 and the power source 202 are electrically coupled to the functional unit 212 by the single pair of electric connection paths 214, 216. In particular the control circuitry 204 is electrically connected to one terminal of the power source 202 and to the first path 214 in the single pair of electrical connection paths 214, 216. The second path 216 in the single pair of electric connection paths 214, 216 is connected to ground and the other terminal of the power source 202. The control circuitry 204 is also connected to the second path 216. In other embodiments, the second path 216 may be connected to the circuitry comprised within the main body 206 in an alternative manner. For example, in some embodiments, the second path 216 may not be connected to ground.

In this illustrated embodiment, the control circuitry 204 and the power source 202 are configured to switch between providing first and second voltages to the functional unit 212 via the single pair of electric connection paths 214, 216. The first voltage exceeds the threshold voltage of the functional member 208, so that the functional member 208 is activated and performs the required function. The second voltage is below the threshold voltage of the functional member 208, so that the functional member 208 is not active and the control circuitry 204 is enabled to obtain a measurement signal from the sensing component 210 via the single pair of electric connection paths 214, 216.

It will be appreciated that configuring the functional unit 212 in such a manner (that is, such that the functional member 208 may both be powered and one or more parameters related to the functional unit 212 may be sensed via the single pair of electric connection paths 214, 216) simplifies the construction of the functional unit 212, the main body 206, and the device 200 itself, particularly where the device 200, or at least the main body 206, is to be waterproof. This is because the same pair of electric connection paths are utilised for the two aforementioned functions, avoiding the need to provide additional electric connections to obtain the measurement signal within the structure of the device 200.

Further example implementations of how a device may be configured in order to provide first and second voltages to a functional unit 212 via a single pair of electric connection paths (and thereby allow the functional unit to both be powered, and one or more parameters related to the functional unit to be sensed via the single pair of electric connection paths) are now described.

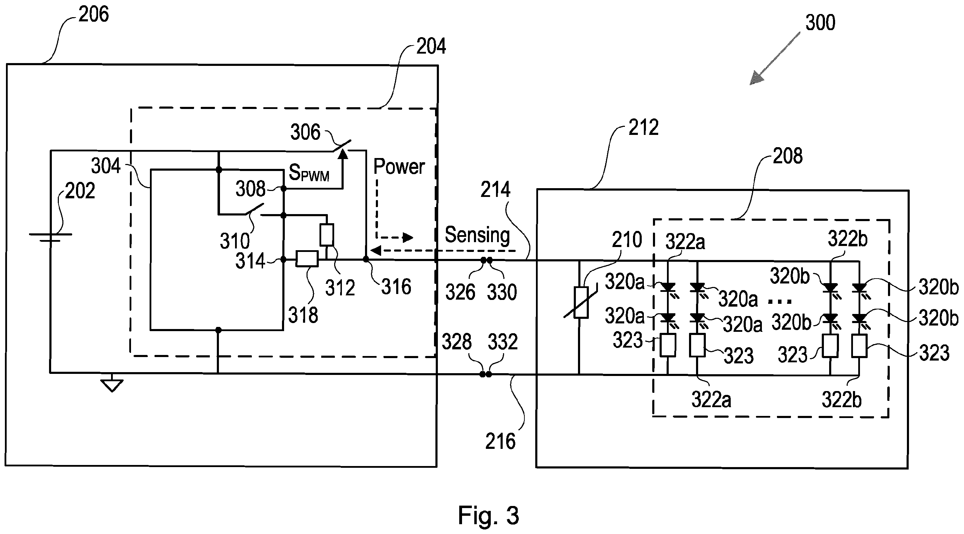

Fig. 3 is a circuit diagram of an exemplary device 300 according to the invention. It will be appreciated that the device 300 is an example implementation of the device 200 described above. It will also be appreciated that the device 300 may be implemented in the form of the electric shaver 1 described above. Elements and components in common to both of Figs. 2 and 3 use the same reference numerals.

In this illustrated embodiment, the control circuitry 204 comprises a control unit 304 that controls the operation of a first switch 306 that is coupled between the power source 202 (for example, a battery) and the first path 214. The control unit 304 controls the first switch 306 using a control signal output from a switch control signal port 308. In some embodiments, the first switch 306 is controlled by a pulse-width modulated control signal, $S_{PWM}$. That is, the first switch 306 is controlled to open and close according to the respectively high/low amplitudes of the $S_{PWM}$ signal. However, it will be appreciated that in other embodiments, the first switch 306 may be controlled via other types of control signal.

The illustrated control circuitry 204 also comprises a second switch 310 (which in this embodiment is shown as part of the control unit 304, although the second switch 310 can alternatively be external to the control unit 304) and a divider resistor 312 that are connected in series between the power source 202 and the first path 214. In alternative embodiments, the second switch 310 can be omitted.

In embodiments in which the second switch 310 is present, the control unit 304 is configured to open and close the second switch 310 to selectively connect/disconnect the divider resistor 312 to the power source 202 and the first path 214. The control unit 304 can control the second switch 310 using the same control signal, or an inverted version of the control signal, output to the first switch 306 from the switch control signal port 308. In some embodiments, the second switch 310 is controlled by a respective pulse-width modulated control signal. The control unit 304 is configured to open the second switch 310 when the first switch 306 is closed, and to open the first switch 306 when the second switch 310 is closed.

The control signal may be such that the first switch 306 is open for approximately the same amount of time that the first switch 306 is closed. However, if the parameter to be measured by the sensing component 210 changes relatively slowly, then it may not be necessary to frequently measure the parameter. In that case, the control signal can be configured such that the first switch 306 is closed for a longer period of time than when the first switch 306 is open.

The control unit 304 also comprises a sensor input channel 314 that is connected to the first path 214 at a connection point 316 between the divider resistor 312 and the sensing component 210. The sensor input channel 314 is used to obtain the measurement signal from the sensing component 210. A second resistor 318 is connected between the sensor input channel 314 and the connection point 316. The second resistor 318 acts to prevent an excessive voltage being received at the control circuitry 204 via the sensor input channel 314 and/or prevent electrostatic discharge disturbances. In some embodiments, the second resistor 318 may be omitted.

As described above with reference to Fig. 2, the functional unit 212 comprises a functional member 208 and a sensing component 210 connected in parallel to each other between the single pair of electric connection paths 214, 216. In this illustrated embodiment, the sensing component 210 is for measuring the temperature of the functional member 208, and is a thermistor, i.e. a component whose resistance changes with changes in temperature.

The functional member 208 comprises a plurality of LEDs 320 connected between the single pair of electric connection paths 214, 216. In Fig. 3 the LEDs 320 are arranged in a number of 'strings' 322, with each string 322 comprising two LEDs 320 and a resistor 323 connected in series. The resistor 323 is used to control the current in the string 322. However, it will be appreciated that this multiple 'string' arrangement is optional, and the LEDs can be arranged in individual strings or in a single string. In this illustrated embodiment, four strings 322 are present in the functional unit 212, with the two strings 322a comprising IR light emitting LEDs 320a, and the two strings 322b comprising visible light emitting LEDs 320b. In this illustrated embodiment, the visible LEDs 320b are activated together with the IR LEDs 320a to indicate activation of the IR function of the functional unit 20. In this embodiment, the four IR LEDs 320a are respectively arranged in a respective one of the central section 34 and the three peripheral sections 35a, 35b, 35c of the skin-contacting surface 32 of the skin heating unit 30 described with reference to Fig. 1, and also the four visible LEDs 320b are respectively arranged in a respective one of the central section 34 and the three peripheral sections 35a, 35b, 35c. However, it will be appreciated that in other embodiments, the number of strings 322a, 322b and the number of IR LEDs 320a and visible LEDs 320b per string 322a, 322b present in the functional unit 212 may be scaled accordingly (for example, depending on the personal care function(s) and/or sensorial experience(s) to be provided by the functional unit 212). The LEDs 320 each have a respective threshold voltage below which the LEDs are electrically non-conductive or substantially electrically non-conductive, and therefore the LEDs do not generate light when the respective voltage across them is less than their threshold voltage. The arrangement of the LEDs 320 and their respective threshold voltages will determine the overall threshold voltage of the functional unit 212.

As noted above, in some embodiments the functional unit 212 is releasably coupled to the main body 206. In this case, the single pair of electrical connection paths 214, 216 between the main body 206 and the functional unit 212 are separable. For this purpose, a first pair of electric connection elements 326, 328 are arranged on the main body 206 that terminate the main body side of the first path 214 and the second path 216 respectively. A pair of electric connection elements 330, 332 are arranged on the functional unit 212 that terminate the functional unit side of the first path 214 and second path 216 respectively. The electric connection elements 326, 328, 330, 332 can comprise any element suitable for establishing an electrical connection when the main body 206 and the functional unit 212 are coupled together. For example the electric connection elements 326, 328, 330, 332 can be conductive plates, plugs/sockets, etc. (thereby enabling a releasable electrical coupling between the functional unit 212 and the main body 206).

It will be appreciated that, in these embodiments, the functional unit 212 may both be powered, and one or more parameters related to the functional unit 212 may be sensed via a single pair of electric connection paths 214, 216. This enables a more robust releasably coupleable device to be provided, as the number of releasably coupleable electrical connections required by the device has been reduced. It will also be appreciated that such a reduction in the number of releasably coupleable electrical connections reduces the complexity of the design of the device to make it watertight.

In this illustrated embodiment, during a first phase of the pulse-width modulated control signal $S_{PWM}$, the first switch 306 is configured to close, and thereby connect the power source 202 to the first path 214. During this first phase, the second switch 310 (if present) is configured to open or be opened, thereby disconnecting the divider resistor 312 from the first path 214. When the first switch 306 is closed, the power source 202 is able to provide a first voltage, e.g. Vi, across the first path 214 and the second path 216. When the first switch 306 is open (e.g. in a second phase of the pulse-width modulated control signal) and the second switch 310 (if present) is closed, the power source 202 is connected to the first path 214 via the divider resistor 312.

The first voltage $V_1$ exceeds the threshold voltage $V_T$ required for the LEDs 320 to operate and generate light. It will be appreciated that no (or substantially no) electrical conduction will occur over a diode when a voltage that is lower than the respective threshold voltage of that diode is applied to the diode. It will be appreciated that substantially no electrical conduction is considered to be occurring over the strings 322 in circumstances in which a measurement signal that is obtained from the sensing component 210 has not been significantly altered. For example, in circumstances in which the impedance across the strings 322 is, say, twenty times greater than the impedance across the sensing component 210, the strings 322 may be considered as substantially non-conductive. In this example, the measurement signal that is obtained from the sensing component 210 will have an accuracy error of less than 5%.

In this illustrated embodiment, one of the purposes of the functional unit 212 is to provide light and warmth as part of a sensorial experience, and thus the functional member 208 comprise NIR LEDs. However, it will be appreciated that the type of functional member 208 provided within the functional unit 212 may be selected so as to provide one or more personal care functions and/or other sensorial experiences. For example, a visual-light emitting LED may be provided in the functional unit 212 in order to enable a skin treatment function to be provided by the device 300 (for example a particular wavelength of light emitted by a visual-light emitting LED may provide particular skin benefits).

In the above embodiments, the functional member 208 comprises an LED or similar component that has an inherent threshold voltage below which the component does not operate. However, the functional member 208 can alternatively comprise one or more components that do not have an inherent 'switch-on' voltage. For example, in some embodiments, a functional member 208 may comprise an electric power consuming member (i.e. other than an LED) that does not itself have a suitable threshold voltage, electrically arranged in series with a diode or a transistor. In such embodiments, it will be appreciated that the electric power consuming member may (when activated), provide a personal care function and/or sensorial experience. The diode or transistor has a threshold voltage that is required for the diode to conduct or the transistor to activate and enable the activation of the electric power consuming member. In other words, the diode or transistor may be arranged in series with the electric power consuming member in such a manner that, when a voltage that is lower than the threshold voltage is applied to the diode or transistor, electrical conduction is prevented or substantially prevented by the diode or the transistor, and thus the voltage is not applied to the electric power consuming member.

When the first switch 306 is closed and the second switch 310 is open, the first voltage $V_1$ is also applied across the sensing component 210. However, with the second switch 310 open, the control circuitry 204 is not able to obtain a valid measurement signal from the sensing component 210 during this first phase of the pulse-width modulated control signal $S_{PWM}$.

During the second phase of the pulse-width modulated control signal $S_{PWM}$, the first switch 306 is open and the second switch 310 is closed, thereby connecting the power source 202 to the first path 304 via the divider resistor 312.

During the second phase of the pulse-width modulated control signal $S_{PWM}$, the first voltage $V_1$ provided by the power source 202 is divided across the divider resistor 312, and the functional unit 212 via the single pair of electric connection paths 214, 216.

As a result, a voltage $V_F$ is provided across the functional unit 212 via the connection point 316, when the first voltage $V_1$ is divided across the divider resistor 312 and the functional unit 212. The voltage $V_F$ is equal to:

$$V_F = V_1 \left( R_F / (R_F + R_{DR}) \right) \tag{1}$$

where $R_F$ is the resistance of the sensing component 210 of functional unit 212, and $R_{DR}$ is the resistance of the divider resistor 312.

That is, in this illustrated embodiment, the control circuitry 304 is configured to switch a divider resistor 312 into series with the power source 202 and the first path 214 of the single pair of electric connection paths to provide the second voltage $V_F$ to the functional unit 212. The divider resistor 312 is configured such that its resistance $R_{DR}$ is sufficiently large enough to result in the value of $V_F$ falling below the aforementioned threshold voltage $V_T$ so that the functional member 208 is non-conductive or substantially non-conductive. In that case, the resistance of the sensing component 210 primarily determines the voltage $V_F$ at the connection point 316 and the sensor input channel 314.

As noted above, the sensing component 210 can be configured to sense an operating parameter of a functional member 208 (for example, one or more of the NIR LEDs 320). For example, the operating parameter may comprise one or more of: a temperature of a functional member 208 or a particular portion of the functional member 208, and a change in a temperature of a functional member 208.

In this illustrated embodiment, the sensing component 210 comprises a thermistor. That is, the sensing component 210 comprises a temperature dependent resistor 210 with a known transfer characteristic, in which a resistance value of the temperature dependent resistor 210 corresponds to a known temperature value. It will be appreciated that this thermistor resistance value may be converted to a corresponding temperature value by means of a mathematical expression (based on one or more properties of the sensing component), or by means of a look-up table. It will also be appreciated that, in other embodiments, other types of sensing component 210 that sense additional or alternative parameters to temperature may be utilised in the device 300.

Therefore, in the second phase of the pulse-width modulated control signal $S_{PWM}$, the control circuitry 204 obtains a measurement signal (the voltage $V_F$) from the sensing component 210 via the sensor input channel 314. As noted above, with the divider resistor 312 in series with the power source 202 and the first path 214 of the single pair of electric connection paths (for example when the second switch 310 - if present - is closed), the measurement signal may be obtained from the sensing component 210 via the sensor input channel 314.

In embodiments where the second resistor 318 is present, the measurement signal received at the sensor input channel 314 represents the second voltage $V_F$ via the second resistor 318. As noted above, the second resistor 318 may prevent an excessive voltage being received at the control circuitry 204 via the sensor input channel 314 and/or prevent Electrostatic Discharge disturbances.

The control circuitry 204 may then, based on the obtained measurement signal, the known resistance of the divider resistor, the known resistance of the second resistor 318 (if present), and the known value of the first voltage Vi, determine the resistance $R_F$ of the sensing component 210 according to Equation (1). The control circuitry 204 may then convert this determined resistance $R_F$ into a corresponding temperature value (relating to the temperature of one or more LEDs 320 and/or relating to the temperature of the functional unit 212) by means of a mathematical expression or by means of a look-up table, for example.

It will be appreciated that the control circuitry 204 may be further configured to control the first voltage $V_1$ in dependence on the obtained measurement signal. That is, the control circuitry 204 may be further configured to provide closed-loop control (via variation of the first voltage Vi) of the functional unit 212 and/or the functional member 208 in accordance with the value of the sensed parameter. For example, the control circuitry 204 may, in circumstances in which it is determined that the sensed temperature of the functional member 208 exceeds a first value, lower the value of the first voltage $V_1$ (but still above the threshold voltage) to reduce the electric power consumption of the functional member 208, and reduce the temperature of the functional member 208. Here, the reduced value of the first voltage $V_1$ will still be above the threshold value to ensure that the functional member 208 continues to operate. In another example, the control circuitry 204 may lower the duty cycle of the $S_{PWM}$ signal to reduce the electric power consumption of the functional member 208, and reduce the temperature of the functional member 208.

Additionally, in some embodiments, based on the obtained measurement signal, the control circuitry 204 may be configured to disconnect the power source 202 from the functional unit 212 or otherwise deactivate the functional member 208. It will be appreciated this may be implemented in circumstances in which the value of the sensed temperature indicates a temperature of a functional member 208 has exceeded a maximum threshold, and poses a risk to a user of the device or to the device itself.

It will be appreciated that, in other embodiments, the control circuitry 204 and the power source 202 may be configured to switch between providing a first voltage and a second voltage via a single switch (as opposed to two switches 306, 310). For example, the control circuitry 204 may comprise one switch having an input and first and second outputs. The input of the switch is connected to the power source 202, the first output of the switch is connected to the first path 214, and the second output of the switch is connected to the first path 214 via the divider resistor 312. In these embodiments, the control circuitry 204 may then be configured to operate the switch to switch between the first output to provide the first voltage to the functional unit 212 to operate the functional member 208, and the second output to provide the second voltage to the functional unit 212 to obtain the measurement signal. For example, this operation of a single switch may be controlled via a pulse wave modulated control signal (in a similar manner to the first switch 306 as described above).

As noted above, it will be appreciated that a functional member 208 that has a threshold voltage below which the functional member is electrically non-conductive or substantially electrically non-conductive, and above which the functional member is electrically conductive may be implemented in an alternative manner to that described with reference to Fig. 3. In particular, these alternative arrangements provide a high impedance when the second voltage is applied in order to obtain the measurement signal from the sensing component 210.

That is, it is not necessary to provide a diode as part of the functional member in order to implement the aforementioned functionality (in addition to providing one or more further elements to provide one or more personal care functions and/or sensorial experiences). Two examples of such an alternative implementation, comprising a transistor in the form of a MOSFET, is now described below.

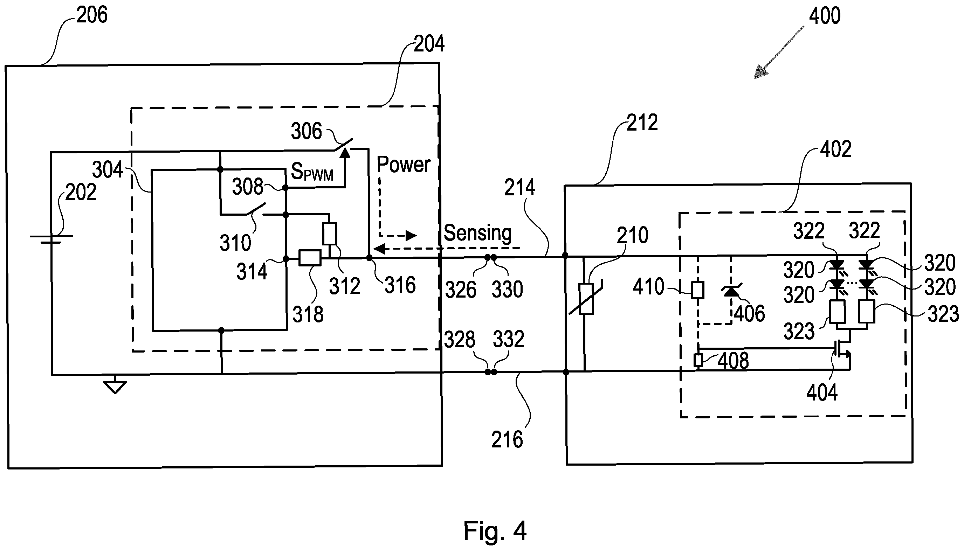

Fig. 4. is a circuit diagram of an exemplary device 400. It will be appreciated that the device 400 is an example implementation of the device 200 described above. It will also be appreciated that the device 400 may be implemented in the form of the electric shaver 1 described above. Elements of the device 400 shown in Fig. 4 that are the same as elements of the device 300 shown in Fig. 3 are indicated by the same reference numerals and are not described further herein.

In this illustrated embodiment, the threshold voltage below which the functional member 402 is electrically non-conductive or substantially electrically non-conductive is the threshold voltage of a transistor 404 (e.g. a MOSFET transistor). That is, it is the voltage that is provided to the gate of the MOSFET 404 that will determine whether the rest of the functional member 402 is activated or not. Two ways in which the voltage that is to be provided to the MOSFET 404 of a functional member 402 may be controlled are described below.

The functional member 402 comprises two strings 322 comprising a respective pair of LEDs 320 and a resistor 323 as described above with reference to Fig. 3. In contrast to Fig. 3 however, the MOSFET 404 is connected between the strings 322 and the second path 216.

Fig. 4 illustrates two alternative implementations for controlling the voltage that is to be provided to the gate of the MOSFET 404 of the functional member 402.

The first implementation uses a Zener diode 406 connected to the first path 214 and arranged in series with a third resistor 408 that is connected to the second path 216. The gate of the MOSFET 404 is connected between the Zener diode 406 and the third resistor 408. The second implementation uses a fourth resistor 410 that is connected to the first path 214 and arranged in series with the third resistor 408 that is connected to the second path 216. The gate of the MOSFET 404 is connected between the third resistor 408 and the fourth resistor 410.

With reference to the first implementation, as noted above, when the first switch 306 is closed, the first voltage $V_1$ is provided across the Zener diode 406 and the third resistor 408. The first voltage $V_1$ exceeds a threshold voltage $V_T$ that in this embodiment is determined by the Zener diode 406, of the MOSFET 404 and the resistance of the third resistor 408), and thus the MOSFET 404 is switched on allowing the first voltage $V_1$ to activate the LEDs 320 to generate light. When the first switch 306 is open and the second switch 310 (if present) is closed, the second voltage is applied across the Zener diode 406 and the third resistor 408, but this voltage is insufficient to activate the MOSFET 404, so the LEDs 320 are inactive and the functional member 402 is electrically non-conductive (or substantially electrically non-conductive), enabling the control unit 304 to obtain the measurement signal.

With reference to the second implementation, as noted above, when the first switch 306 is closed, the first voltage $V_1$ is provided across the fourth resistor 408 and the third resistor 410 which form a voltage divider. The value of the voltage that will be provided to the MOSFET 404 from the voltage divider will be sufficient to activate the MOSFET 404, thereby allowing electrical conduction across the strings 322, activating the NIR LEDs 320.

When the first switch 306 is open and the second switch 310 (if present) is closed, the second voltage $V_F$ is provided across the voltage divider formed from the third resistor 408 and the fourth resistor 410. The voltage output by the voltage divider and provided to the MOSFET 404 will be insufficient to activate the MOSFET 404, thereby preventing electrical conduction across the strings 322.

It will be appreciated that both the aforementioned implementations provide a high impedance across the functional member 402 during sensor reading using the second voltage. The measurement signal can be evaluated in a similar way to that described above with reference to Fig. 3 to determine the sensed parameter (e.g. temperature).

The first implementation provides a high impedance across the functional member 402 when the voltage applied to the functional unit 212 does not exceed the threshold voltage $V_T$.

The second implementation provides a high impedance across the functional member 402 when the voltage applied to the functional unit 212 does not activate the MOSFET 410 following the voltage drop between the third resistor 408 and the fourth resistor 410.

In some embodiments, the functional unit 212 may comprise more than one type of functional member that are to be operated separately. In these embodiments, the functional unit 212 may be capable of providing a plurality of different personal care functions and/or sensorial experiences that are associated with each of the different types of functional members. An embodiment comprising two types of functional members with respective voltage requirements is now described below.

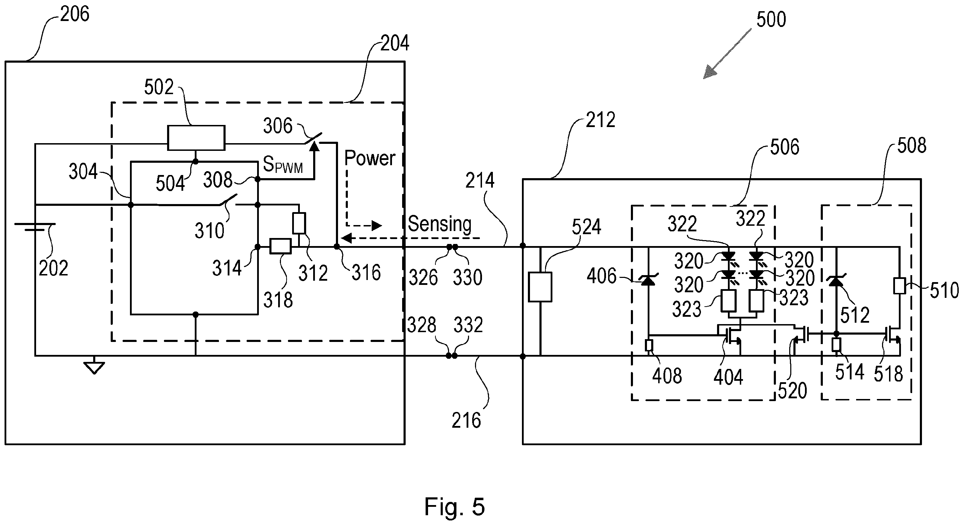

Fig. 5. is a circuit diagram of an exemplary device 500. It will be appreciated that the device 500 is an example implementation of the device 200 described above. It will also be appreciated that the device 500 may be implemented in the form of the electric shaver 1 described above. Elements of the device 500 shown in Fig. 5 that are the same as elements of the devices 300 and 400 shown in Fig. 3 and Fig. 4 respectively are indicated by the same reference numerals and are not described further herein.

In this illustrated embodiment, to generate different voltage levels from the power source 202 for use by different types of functional members, a DC/DC converter 502 is disposed between the power source 202, and the first switch 306. The DC/DC converter 502 is configured to generate two or more voltages from the voltage provided by the power source 202 that is then provided to the first path 214 via the first switch 306, when the first switch 306 is closed.

The DC/DC converter 502 is configured to generate two or more voltages in accordance with a control signal provided by an output 504 of the control circuitry 204. Output 504 is also referred to as 'setV' output 504.

The DC/DC converter 502 is configured to selectively provide a voltage of at least a first activation voltage $V_{A1}$, and at a second activation voltage $V_{A2}$ to the functional unit 212 to respectively activate a first functional member 506 and a second functional member 508.

The first functional member 506 corresponds to the Zener diode-based implementation of the functional member 402 in Fig. 4. Thus, the first functional member 506 comprises a first set of NIR LEDs 320 for providing a heating effect to the user. The Zener diode 406 is referred to below as Z1, and MOSFET 404 is referred to as M1.

The second functional member 508 comprises an electric power consuming member 510 for providing a different personal care function or sensory experience to the user than the LEDs 320 in the first functional member 506. The electric power consuming member 510 may, for example, comprise one or more blue light emitting LEDs (for example, to treat acne). In another example, the electric power consuming member 510 may comprise one or more visual light emitting LEDs (for example, for user interface purposes).

The function provided by the second functional member 508, and the function provided by the first functional member 506 may be provided independently of one another.

In this illustrated embodiment, the second functional member 508 is controlled using an arrangement of a Zener diode, resistor and MOSFET similar to the first functional member 506. Thus, the second functional member comprises a second Zener diode 512 (referred to as Z2) connected to the first path 214 and arranged in series with a fifth resistor 514 that is connected to the second path 216. The gate of the MOSFET 518 (referred to as M2) is connected between Zener diode 512 and the fifth resistor 514. The electric power consuming member 510 is connected between the first path 214 and the source terminal of the MOSFET 518.

A further MOSFET 520 (referred to as M3) is also provided in the functional unit 212. The source terminal of M3 520 is connected to the gate of M1 404. The gate of M3 520 is connected between Z2 512 and the fifth resistor 514 and thus also to the gate of M2 518. The drain terminal of M3 520 is connected to the second path 216.

The first functional member 506 is configured so that the LEDs 320 are activated when a voltage is provided from the DC/DC converter 502 that is above a first threshold value (also referred to as VZ1, a threshold related to Zener diode 406 and the MOSFET 404) and below a second threshold value (also referred to as VZ2, a threshold related to Zener diode 512 and including the MOSFET 518). The second functional member 508 is configured so that the electric power consuming member 510 is activated when a voltage is provided from the DC/DC converter 502 that is above the second threshold value VZ2. When the voltage from the control circuitry 304 is below the first threshold value VZ1 (i.e. the second voltage provided the second switch 310 and the divider resistor 312), a measurement signal can be obtained from sensing component 524 that is connected between the first path 214 and the second path 216. The sensing component 524 may be a thermistor as described above, or a different type of sensing component. In some embodiments, the sensing component 524 can be a sensor circuit that includes one or more sensors.

When the first switch 306 is closed and the first activation voltage $V_{A1}$ is provided to the functional unit 212 via the single pair of electric connection paths 214, 216, the first activation voltage $V_{A1}$ is provided across the components of the functional unit 212. The first activation voltage $V_{A1}$ exceeds the threshold voltage VZ1 of Zener diode 406, and thus the first Zener diode 406 is electrically conductive under the application of the first activation voltage $V_{A1}$.

The first activation voltage is sufficient to activate M1 404, thereby allowing electrical current to flow through the strings 322 in a similar manner as described above. That is, the first functional member 506 will be activated under the application of the first activation voltage $V_{A1}$ to the functional unit 212. As the first activation voltage is below the second Zener threshold VZ2, the second functional member 508 is not activated.

When the first switch 306 is closed and the second activation voltage $V_{A2}$ is provided to the functional unit 212 via the single pair of electric connection paths 214, 216, the second activation voltage $V_{A2}$ is provided across the components of the functional unit 212. The second activation voltage $V_{A2}$ exceeds the threshold voltage VZ2 of Zener diode 512, and thus the Zener diode 512 is electrically conductive under the application of the second activation voltage $V_{A2}$.

The second activation voltage is sufficient to activate M2 518, thereby allowing electrical current to flow through the electric power consuming member 510, and activating the function provided by the electric power consuming member 510. That is, the second functional member 508 will be activated under the application of the second activation voltage $V_{A2}$ to the functional unit 212. As the second voltage level is also above the first Zener threshold VZ1, the Zener diode Z1 406 is also conductive, but the MOSFET M3 520 causes MOSFET M1404 to remain off and prevent current flowing through the LEDs 320.

Therefore, in this illustrated embodiment, the functional unit is configured in such a manner that both the first functional member 506 and the second functional member 508 may be independently activated. However, in other embodiments, it will be appreciated that the functional unit 212 may be arranged in such a manner that both the first and second functional members 506, 508 may be activated under the application of the second activation voltage $V_{A2}$ (for example, by removing MOSFET M3 520).

It will also be appreciated that the first and second Zener diodes 406, 512 may be replaced by a voltage divider arrangement as described with reference to Fig. 4.

With reference now to when the second switch 310 is closed (and the first switch 306 is open), the aforementioned second voltage $V_F$ (which is less than VZ1) will be provided across the sensing component 524 between the first path 214 and the second path 216.

As the second voltage $V_F$ does not exceed the threshold voltage VZ1 of the first Zener diode 406, the first Zener diode 508 and the second Zener diode 512 are not electrically conductive and neither functional member 506, 508 will be activated.

Therefore, the resistance of the sensing component 524 and the divider resistor 312 will determine the voltage of the measurement signal obtained from the sensing component 524. The control circuitry 204 may determine the value of the resistance of the sensing component 524 and the relevant metric or parameter represented by the resistance in accordance with the methods above.

In embodiments where the sensing component 524 is a sensor circuit, the sensor circuit 524 may comprise one or more sensors. In these embodiments, rather than the resistance of the sensing component 524 indicating the measured parameter, the measurements by the one or more sensors may be amplitude modulated on to the second voltage signal. The control circuitry 204 may then decode (demodulate) the amplitude modulated measurement signal received at the sensor input channel 314 in order to determine the parameter measurement(s). In some embodiments, the identity of the sensor that obtained the measurement(s) may also be amplitude modulated on to the second voltage and thus the control circuitry 204 can identify the one or more sensors that have obtained measurements and determine the respective sensor measurements for each of those sensors.

Therefore there is provided a device comprising a functional unit and a main body that enables the functional unit to be powered and a measurement signal to be obtained from the functional unit via a single pair of electrical connection paths.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1.  A device (200, 300, 400, 500) comprising:

    a main body (206) comprising a power source (202) and control circuitry (204); and
    a functional unit (212) comprising a functional member (208, 402) and a sensing component (210, 524), wherein the functional member (208, 402) and the sensing component (210, 524) are arranged electrically in parallel with one another, and wherein the functional member (208, 402) has a threshold voltage below which the functional member (208, 402) is electrically non-conductive or substantially electrically non-conductive, and above which the functional member (208, 402) is electrically conductive,
    wherein the control circuitry (204) and the power source (202) are electrically coupled to the functional unit (212) by a single pair of electric connection paths (214, 216),
    and wherein the control circuitry (204) and the power source (202) are configured to switch between providing first and second voltages to the functional unit (212) via the single pair of electric connection paths (214, 216), wherein the first voltage exceeds the threshold voltage and the second voltage is below the threshold voltage to enable a measurement signal to be obtained from the sensing component (210, 524) via the single pair of electric connection paths (214, 216).

2.  The device (200, 300, 400, 500) according to claim 1, wherein, by providing the first voltage to the functional unit (212) via the single pair of electric connection paths (214, 216), the control circuitry (204) and the power source (202) activate the functional member (208, 402).

3.  The device (200, 300, 400, 500) according to claim 1 or 2, wherein the functional unit (212) is releasably coupled to the main body (206), and wherein the single pair of electric connection paths (214, 216) comprises a first pair of electric connection elements (326, 328) arranged on the main body (206), and a second pair of electric connection elements (330, 332) arranged on the functional unit (212), wherein the second pair of electric connection elements (330, 332) are releasably coupled to the first pair of electric connection elements (326, 328).

4.  The device (200, 300, 400, 500) according to any of claims 1-3, wherein the functional member (208, 402) comprises one or more light emitting diodes.

5.  The device (200, 300, 400, 500) according to any of claims 1-3, wherein the functional member (208, 402) comprises an electric power consuming member (510) electrically arranged in series with a diode or a transistor.

6.  The device (200, 300, 400, 500) according to any preceding claim, wherein the sensing component (210, 524) is configured to sense an operating parameter of the functional member (208, 402).

7.  The device (200, 300, 400, 500) according to claim 6, wherein the operating parameter is one or more of: a temperature of the functional member (208, 402), and a change in a temperature of the functional member (208, 402).

8.  The device (200, 300, 400, 500) according to any of claims 1-7, wherein the sensing component (210, 524) comprises a thermistor.

9. The device (200, 300, 400, 500) according to any of claims 1-8, wherein the control circuitry (204) is configured to close a first switch (306) to connect the power source (202) directly to a first path (214) of the single pair of electric connection paths (214, 216) to provide the first voltage, and configured to provide the second voltage to the first path (214) when the first switch (206) is open using a divider resistor (312) in series with the power source (202) and the first path (214) of the single pair of electric connection paths (214, 216).

10. The device (200, 300, 400, 500) according to claim 9, wherein the control circuitry (204) comprises a sensor input channel that is connected to the first path (214) at a connection point (316) between the divider resistor (312) and the sensing component (210, 524) to obtain the measurement signal from the sensing component (210, 524).

11. The device (200, 300, 400, 500) according to claim 10, wherein the control circuitry (204) further comprises: a second switch (310) arranged in series with the divider resistor (312), the power source (202) and the first path (214), wherein the second switch (310) and the divider resistor (312) are also arranged in parallel to the first switch (306), and wherein the control circuitry (204) is configured to: close the second switch (310) while the first switch (306) is open to provide the second voltage to the functional unit (212) and to obtain the measurement signal from the sensing component (210, 524) via the sensor input channel (314).

12. The device (200, 300, 400, 500) according to claim 11, wherein the control circuitry (204) is configured to open and close the first switch (306) and/or the second switch (310) according to a pulse-width modulated control signal.

13. The device (200, 300, 400, 500) according to any of claims 1-8, wherein the control circuitry (204) further comprises: a switch having an input and first and second outputs, wherein the input of the switch is connected to the power source (202), the first output of the switch is connected to a first path (214) of the single pair of electric connection paths (214, 216), and the second output of the switch is connected to the first path (214) via a divider resistor (312), and wherein the control circuitry (204) is configured to: operate the switch to switch between the first output to provide the first voltage and the second output to provide the second voltage.

14. The device (200, 300, 400, 500) according to any of claims 1-13, wherein the device (200, 300, 400, 500) is a personal care device, and wherein the functional unit (212) is a personal care unit.

15. The device (200, 300, 400, 500) according to claim 14, wherein:

   the personal care device is an electric shaver (1);
   the functional unit (212) is a shaving unit (20) releasably coupled to the main body (10) and comprising one or more hair-cutting units (21); and
   the functional member (208, 402) comprises one or more light emitting diodes (LEDs) arranged in the shaving unit (20) to emit light during operation of the one or more hair-cutting units (21).

**Patentansprüche**

1. Vorrichtung (200, 300, 400, 500), umfassend:

   einen Hauptkörper (206), der eine Stromquelle (202) und eine Steuerschaltung (204) umfasst; und eine Funktionseinheit (212), die ein Funktionselement (208, 402) und eine Sensorkomponente (210, 524) umfasst, wobei das Funktionselement (208, 402) und die Sensorkomponente (210, 524) elektrisch parallel zueinander angeordnet sind und wobei das Funktionselement (208, 402) eine Schwellenspannung aufweist, unterhalb derer das Funktionselement (208, 402) elektrisch nicht leitend oder im Wesentlichen elektrisch nicht leitend ist und oberhalb derer das Funktionselement (208, 402) elektrisch nicht leitend ist elektrisch leitfähig, wobei die Steuerschaltung (204) und die Stromquelle (202) über ein einzelnes Paar elektrischer Verbindungspfade (214, 216) elektrisch mit der Funktionseinheit (212) verbunden sind, und wobei die Steuerschaltung (204) und die Stromquelle (202) so konfiguriert sind, dass sie zwischen der Bereitstellung einer ersten und einer zweiten Spannung an die Funktionseinheit (212) über das einzelne Paar elektrischer Verbindungspfade (214, 216) umschalten, wobei die erste Spannung die Schwellenspannung überschreitet und die zweite Spannung unter der Schwellenspannung liegt, um zu ermöglichen, dass ein Messsignal von der Erfassungskomponente (210, 524) über das einzelne Paar elektrischer Verbindungspfade (214, 216) erhalten wird.

2. Vorrichtung (200, 300, 400, 500) nach Anspruch 1, wobei die Steuerschaltung (204) und die Stromquelle (202) das Funktionselement (208, 402) aktivieren, indem sie der Funktionseinheit (212) über das einzelne Paar elektrischer Verbindungswege (214, 216) die erste Spannung zuführen.

3. Vorrichtung (200, 300, 400, 500) nach Anspruch 1 oder 2, wobei die Funktionseinheit (212) lösbar mit dem Hauptkörper (206) gekoppelt ist und wobei das einzelne Paar elektrischer Verbindungspfade (214, 216) umfasst ein erstes Paar an dem Grundkörper (206) angeordneter elektrischer Anschlusselemente (326, 328) und ein zweites Paar an an der Funktionseinheit (212) angeordneter elektrischer Anschlusselemente (330, 332), wobei das zweite Paar von elektrischen Verbindungselementen (330, 332) lösbar mit dem ersten Paar elektrischer Verbindungselemente (326, 328) gekoppelt sind.

4. Vorrichtung (200, 300, 400, 500) nach einem der Ansprüche 1-3, wobei das Funktionselement (208, 402) eine oder mehrere Leuchtdioden umfasst.

5. Vorrichtung (200, 300, 400, 500) nach einem der Ansprüche 1-3, wobei das Funktionselement (208, 402) ein elektrische Energie verbrauchendes Element (510) umfasst, das elektrisch in Reihe mit einer Diode oder einem Transistor angeordnet ist.

6. Vorrichtung (200, 300, 400, 500) nach einem der vorhergehenden Ansprüche, wobei die Erfassungskomponente (210, 524) zum Erfassen eines Betriebsparameters des Funktionselements (208, 402) konfiguriert ist.

7. Vorrichtung (200, 300, 400, 500) nach Anspruch 6, wobei der Betriebsparameter einer oder mehrere von Folgendem ist: eine Temperatur des Funktionselements (208, 402) und eine Änderung einer Temperatur des Funktionselements (208, 402).

8. Vorrichtung (200, 300, 400, 500) nach einem der Ansprüche 1-7, wobei die Sensorkomponente (210, 524) einen Thermistor umfasst.

9. Vorrichtung (200, 300, 400, 500) nach einem der Ansprüche 1-8, wobei die Steuerschaltung (204) so konfiguriert ist, dass sie einen ersten Schalter (306) schließt, um die Stromquelle (202) direkt mit einem zu verbinden ersten Pfad (214) des einzelnen Paars elektrischer Verbindungspfade (214, 216), um die erste Spannung bereitzustellen, und dazu konfiguriert ist, die zweite Spannung an den ersten Pfad (214) bereitzustellen, wenn der erste Schalter (206) unter Verwendung eines Teilers geöffnet ist Widerstand (312) in Reihe mit der Stromquelle (202) und dem ersten Pfad (214) des einzelnen Paares elektrischer Verbindungspfade (214, 216).

10. Vorrichtung (200, 300, 400, 500) nach Anspruch 9, wobei die Steuerschaltung (204) einen Sensoreingangskanal umfasst, der an einem Verbindungspunkt (316) zwischen dem Teiler mit dem ersten Pfad (214) verbunden ist Widerstand (312) und die Sensorkomponente (210, 524), um das Messsignal von der Sensorkomponente (210, 524) zu erhalten.

11. Vorrichtung (200, 300, 400, 500) nach Anspruch 10, wobei die Steuerschaltung (204) weiterhin umfasst: einen zweiten Schalter (310), der in Reihe mit dem Teilerwiderstand (312), der Stromquelle (202) und dem ersten Pfad (214) angeordnet ist, wobei der zweite Schalter (310) und der Teilerwiderstand (312) ebenfalls parallel angeordnet sind an den ersten Schalter (306), und wobei die Steuerschaltung (204) so konfiguriert ist, dass sie: schließen des zweiten Schalters (310), während der erste Schalter (306) geöffnet ist, um der Funktionseinheit (212) die zweite Spannung bereitzustellen und das Messsignal von der Sensorkomponente (210, 524) über den Sensoreingangskanal (314) zu erhalten.

12. Vorrichtung (200, 300, 400, 500) nach Anspruch 11, wobei die Steuerschaltung (204) konfiguriert ist, um den ersten Schalter (306) und/oder den zweiten Schalter (310) gemäß einer Pulsweitenmodulation zu öffnen und zu schließen Steuersignal.

13. Vorrichtung (200, 300, 400, 500) nach einem der Ansprüche 1-8, wobei die Steuerschaltung (204) außerdem Folgendes umfasst: einen Schalter mit einem Eingang und ersten und zweiten Ausgängen, wobei der Eingang des Schalters mit der Stromquelle (202) verbunden ist und der erste Ausgang des Schalters mit einem ersten Pfad (214) des einzelnen Paars elektrischer Verbindungspfade verbunden ist (214, 216) und der zweite Ausgang des Schalters über einen Teilerwiderstand (312) mit dem ersten Pfad (214) verbunden ist, und wobei die Steuerschaltung (204) dazu konfiguriert ist: betätigen Sie den Schalter, um zwischen dem ersten Ausgang zur Bereitstellung der ersten Spannung und dem zweiten Ausgang zur Bereitstellung der zweiten Spannung umzuschalten.

14. Vorrichtung (200, 300, 400, 500) nach einem der Ansprüche 1-13, wobei das Gerät (200, 300, 400, 500) ein Körperpflegegerät ist und wobei die Funktionseinheit (212) ein persönliche Pflegeeinheit.

15. Vorrichtung (200, 300, 400, 500) nach Anspruch 14, wobei: das Körperpflegegerät ein Elektrorasierer (1) ist; die Funktionseinheit (212) eine Rasiereinheit (20) ist, die lösbar mit dem Hauptkörper (10) gekoppelt ist und eine oder mehrere Haarschneideeinheiten (21) umfasst; und das Funktionselement (208, 402) eine oder mehrere Leuchtdioden (LED) umfasst, die in der Rasiereinheit (20) angeordnet sind, um während des Betriebs der einen oder mehreren Haarschneideeinheiten (21) Licht zu emittieren.

## Revendications

1. Un dispositif (200, 300, 400, 500) comprenant:

   un corps principal (206) comprenant une source d'alimentation (202) et un circuit de commande (204); et une unité fonctionnelle (212) comprenant un élément fonctionnel (208, 402) et un composant de détection (210, 524), l'élément fonctionnel (208, 402) et le composant de détection (210, 524) étant disposés électriquement en parallèle l'un avec l'autre, et dans lequel l'élément fonctionnel (208, 402) a une tension seuil en dessous de laquelle l'élément fonctionnel (208, 402) est électriquement non-conducteur ou sensiblement non-conducteur, et au-dessus de laquelle l'élément fonctionnel (208, 402) est électriquement conducteur, le circuit de commande (204) et la source d'alimentation (202) étant couplés électriquement à l'unité fonctionnelle (212) par une paire unique de chemins de connexion électrique (214, 216), et dans lequel le circuit de commande (204) et la source d'alimentation (202) sont configurés pour commuter entre la fourniture d'une première et d'une seconde tension à l'unité fonctionnelle (212) via la paire unique de chemins de connexion électrique (214, 216), la première tension dépasse la tension de seuil et la seconde tension est inférieure à la tension de seuil pour permettre l'obtention d'un signal de mesure au niveau du composant de détection (210, 524) par l'intermédiaire de la paire unique de chemins de connexion électrique (214, 216).

2. Un dispositif (200, 300, 400, 500) selon la revendication 1, où, en fournissant la première tension appliquée à l'unité fonctionnelle (212) via la paire unique de chemins de connexion électrique (214, 216), le circuit de commande (204) et la source d'alimentation (202) activent l'élément fonctionnel (208, 402) .

3. Un dispositif (200, 300, 400, 500) selon la revendication 1 ou 2, dans lequel l'unité fonctionnelle (212) est couplée de manière amovible au corps principal (206) et dans lequel la paire unique de chemins de connexion électrique (214, 216) comprend une première paire d'éléments de connexion électrique (326, 328) disposés sur le corps principal (206), et une seconde paire d'éléments de connexion électrique (330, 332) disposée sur l'unité fonctionnelle (212), la seconde paire d'éléments de connexion électrique (330, 332) étant couplée de manière libérable à la première paire d'éléments de connexion électrique (326, 328) .

4. Un dispositif (200, 300, 400, 500) selon l'une quelconque des revendications 1 à 3, dans lequel l'élément fonctionnel (208, 402) comprend une ou plusieurs diodes électroluminescentes.

5. Un dispositif (200, 300, 400, 500) selon l'une quelconque des revendications 1 à 3, dans lequel l'élément fonctionnel (208, 402) comprend un élément consommateur d'énergie électrique (510) agencé électriquement en série avec une diode ou un transistor.

6. Un dispositif (200, 300, 400, 500) selon l'une quelconque des revendications précédentes, dans lequel le composant de détection (210, 524) est configuré pour détecter un paramètre de fonctionnement de l'élément fonctionnel (208, 402).

7. Un dispositif (200, 300, 400, 500) selon la revendication 6, dans lequel le paramètre de fonctionnement est un ou plusieurs parmi: une température de l'élément fonctionnel (208, 402), et un changement d'une température de l'élément fonctionnel (208, 402).

8. Un dispositif (200, 300, 400, 500) selon l'une quelconque des revendications 1 à 7, dans lequel le composant de détection (210, 524) comprend une thermistance.

9. Un dispositif (200, 300, 400, 500) selon l'une quelconque des revendications 1 à 8, dans lequel le circuit de commande (204) est configuré pour doser un premier commutateur (306) pour connecter la source d'alimentation (202) directement à un premier trajet (214) de la paire unique de trajets de connexion électrique (214, 216) pour fournir la première tension, et configuré pour fournir la seconde tension au premier chemin (214) lorsque le premier commutateur (206) est ouvert au moyen d'une résistance diviseuse (312) en série avec la source d'alimentation (202) et le premier chemin (214) de la paire unique de chemins de connexion électrique (214, 216).

10. Un dispositif (200, 300, 400, 500) selon la revendication 9, dans lequel le circuit de commande (204) comprend un canal d'entrée de capteur qui est connecté au premier trajet (214) au niveau d'un point de connexion (316) entre la résistance de division (312) et le composant de détection (210, 524) pour obtenir le signal de mesure, utilisez le composant de détection (210, 524).

11. Un dispositif (200, 300, 400, 500) selon la revendication 10, dans lequel le circuit de commande (204) comprend en outre: un second commutateur (310) monté en série avec la résistance de division (312), la source d'alimentation (202) et le premier trajet (214), le second commutateur (310) et la résistance de division (312) étant également montés en parallèle avec le premier commutateur (306), et le circuit de commande (204) étant configuré pour: fermer le second commutateur (310) lorsque le premier commutateur (306) est ouvert pour fournir la seconde tension à l'unité fonctionnelle (212) et pour obtenir le signal de mesure sur le composant de détection (210, 524) via le canal d'entrée de capteur (314).

12. Le dispositif (200, 300, 400, 500) selon la revendication 11, dans lequel le circuit de commande (204) est configuré pour ouvrir et fermer le premier commutateur (306) et/ou le second commutateur (310) selon un signal de commande modulé en largeur d'impulsion.

13. Le dispositif (200, 300, 400, 500) selon l'une quelconque des revendications 1 à 8, dans lequel le circuit de commande (204) comprend en outre: un commutateur ayant une entrée et des premières et secondes sorties, l'entrée du commutateur étant connectée à la source d'alimentation (202), la première sortie du commutateur étant connectée à un premier trajet (214) de la paire unique de trajets de connexion électrique (214, 216), et la seconde sortie du commutateur est connectée au premier chemin (214) par l'intermédiaire d'une résistance diviseuse (312), et dans laquelle le circuit de commande (204) est configuré pour: actionner le commutateur pour commuter entre la première sortie pour fournir la première tension et la seconde sortie pour fournir la seconde tension.

14. Le dispositif (200, 300, 400, 500) selon l'une quelconque des revendications 1 à 13, dans lequel le dispositif (200, 300, 400, 500) est un dispositif de soins personnels, et dans lequel l'unité fonctionnelle (212) est une unité de soins personnels.

15. Le dispositif (200, 300, 400, 500) selon la revendication 14, dans lequel: le dispositif de soins personnels est un rasoir électrique (1); l'unité fonctionnelle (212) est une unité de rasage (20) couplée amovible au corps principal (10) et comprenant une ou plusieurs unités de coupe de cheveux (21); et l'élément fonctionnel (208, 402) comprend une ou plusieurs diodes électroluminescentes (LED) disposées dans l'unité de rasage (20) pour émettre de la lumière pendant le fonctionnement de l'unité de coupe de cheveux (21).

Fig. 1

Fig. 2

Fig. 3

Fig. 4

Fig. 5

**REFERENCES CITED IN THE DESCRIPTION**

**Patent documents cited in the description**

- WO 2015135682 A1